(19) Europäisches Patentamt
European Patent Office
Office européen des brevets

(11) EP 3 079 649 B1

(12) # EUROPEAN PATENT SPECIFICATION

(45) Date of publication and mention of the grant of the patent:
**20.11.2019 Bulletin 2019/47**

(21) Application number: **14805689.8**

(22) Date of filing: **24.10.2014**

(51) Int Cl.:
***A61K 8/29*** *(2006.01)*
***A61K 8/81*** *(2006.01)*
***A61K 8/02*** *(2006.01)*
***A61Q 19/08*** *(2006.01)*
***A61Q 17/04*** *(2006.01)*
***A61K 8/88*** *(2006.01)*
***A61K 8/19*** *(2006.01)*

(86) International application number:
**PCT/JP2014/079001**

(87) International publication number:
**WO 2015/087637 (18.06.2015 Gazette 2015/24)**

(54) **COMPOSITE PARTICLES COMPRISING A METAL-DOPED INORGANIC UV FILTER, AND COMPOSITIONS CONTAINING THEM**

VERBUNDTEILCHEN MIT EINEM METALLDOTIERTEN ANORGANISCHEN UV-FILTER UND ZUSAMMENSETZUNGEN DAMIT

PARTICULES COMPOSITES COMPRENANT UN FILTRE UV INORGANIQUE DOPE PAR UN METAL, ET COMPOSITIONS CONTENANT CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.12.2013 JP 2013255079**

(43) Date of publication of application:
**19.10.2016 Bulletin 2016/42**

(73) Proprietors:
- **L'OREAL**
**75008 Paris (FR)**
Designated Contracting States:
**AT BE BG CH CY CZ DE DK EE ES FI FR GB GR HR HU IE IS IT LI LT LU LV MC MK MT NL NO PL PT RO RS SE SI SK SM TR**
- **Harada, Yasuko**
**Kawasaki-shi, Kanagawa 213-0012 (JP)**
Designated Contracting States:
**AL**
- **Candau, Didier**
**94152 Chevilly-Larue (FR)**
Designated Contracting States:
**AL**
- **Shimizu, Momoko**
**Kawasaki-shi, Kanagawa 213-0012 (JP)**
Designated Contracting States:
**AL**
- **Matsufuji, Shinichi**
**Kawasaki-shi, Kanagawa 213-0012 (JP)**
Designated Contracting States:
**AL**

(72) Inventors:
- **HARADA, Yasuko**
**Kawasaki-shi**
**Kanagawa, 2130012 (JP)**
- **CANDAU, Dider**
**94152 CHEVILLY-LARUE (FR)**
- **SHIMIZU, Momoko**
**Kawasaki-shi**
**Kanagawa 213-0012 (JP)**
- **MATSUFUJI, Shinichi**
**Kawasaki-shi**
**Kanagawa 213-0012 (JP)**

(74) Representative: **Casalonga**
**Casalonga & Partners**
**Bayerstraße 71/73**
**80335 München (DE)**

(56) References cited:
**WO-A1-2012/105059**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- S. H. ABDUL KALEEL ET AL: "Thermal and Mechanical Properties of Polyethylene/Doped-TiO2 Nanocomposites Synthesized Using In Situ Polymerization", JOURNAL OF NANOMATERIALS, vol. 2, no. 4-5, 1 January 2011 (2011-01-01), pages 1-6, XP055163191, ISSN: 1687-4110, DOI: 10.1016/j.polymer.2004.08.018
- SUN L ET AL: "Effects of undoped and manganese-doped zinc oxide nanoparticles on the colour fading of dyed polyester fabrics", CHEMICAL ENGINEERING JOURNAL, ELSEVIER SEQUOIA, LAUSANNE, CH, vol. 147, no. 2-3, 15 April 2009 (2009-04-15), pages 391-398, XP025942751, ISSN: 1385-8947, DOI: 10.1016/J.CEJ.2008.11.032 [retrieved on 2008-11-25]

## Description

### Technical Field

The subject of the present invention concerns composite particles comprising:

i) a matrix containing at least one inorganic and/or at least one organic material and

ii) at least one inorganic UV filter which is doped by at least one transition metal

wherein the composite particles are spherical particles containing a core comprising at least one organic and/or one inorganic material, the said core being covered at least partially by at least one layer of inorganic UV filter which is doped by at least one transition metal.

The subject of the present invention is also a composition containing, in a physiologically acceptable medium, at least the composite particles as defined above.

This composition is for topical use and is more particularly intended for the photoprotection of the skin and/or hair against ultraviolet (UV) radiation.

### Background Art

It is known that light radiation with wavelengths of between 280 nm and 400 nm permits tanning of the human epidermis and that light rays with wavelengths more particularly between 280 and 320 nm, known as UV-B rays, cause skin burns and erythema which can harm the development of a natural tan.

For these reasons, and also for aesthetic reasons, there is constant demand for means for controlling this natural tanning in order thus to control the colour of the skin; UV-B radiation should thus be screened out.

It is also known that UV-A rays with wavelengths between 320 and 400 nm, which cause tanning of the skin, are liable to induce adverse changes therein, in particular in the case of sensitive skin or skin that is continually exposed to solar radiation. UV-A rays cause in particular a loss of elasticity of the skin and the appearance of wrinkles leading to premature ageing of the skin.

Thus, for aesthetic and cosmetic reasons, for instance conservation of the skin's natural elasticity, people increasingly wish to control the effect of UV-A rays on their skin. It is thus desirable to also screen out UV-A radiation.

There are many cosmetic products comprising one or more inorganic and/or organic UV-screening agents. Fine inorganic particles based on a metal oxide such as titanium dioxide ($TiO_2$) are usually used for children's skin or sensitive skin, to protect the skin against UV rays.

These fine metal oxide particles generally have a mean elementary particle size of less than or equal to 0.1 $\mu$m, preferably between 0.005 and 0.1 $\mu$m, more preferably between 0.01 and 0.1 $\mu$m, and preferentially between 0.015 and 0.05 $\mu$m. However the UVA and UVB filtering efficacies of these particles are also limited.

There are also cosmetic products comprising one or more spherical and/or non-spherical screening composite particles which have a particle size of more than 0.1 $\mu$m and which are described in the following patent applications: WO2012105723, WO2012105060, WO2012105059, WO2011016140, WO2011016143, FR2971152, WO2012104160 and WO2012104161. However the UVA and UVB filtering efficacies of these composites are also limited. Furthermore, their cosmetic properties during application are not completely satisfactory in particular in view of the smooth feeling during spreading of the composition onto the skin which may be translated as a greasy feeling and/or sticky feeling.

There are also metal oxide particles doped by a transition metal which have a mean elementary particle size from 0.001 to 0.5 $\mu$m and which are described in the following patent applications: WO9960994, WO0140114, and WO2005/002538. Their cosmetic properties during application are also not completely satisfactory in particular in view of the smooth feeling after application of the composition onto the skin which may be translated as a greasy feeling and/or sticky feeling.

### Summary of Invention

### Technical Problem

There is thus still a need to find new UV screening materials containing an inorganic UV-filter, which afford more efficient photoprotection both in UVA and UVB and which do not have the drawbacks presented above.

**Solution to Problem**

Unexpectedly and advantageously, the inventors have shown that this need can be met by means of new composite particles comprising:

i) a matrix containing at least one inorganic and/or at least one organic material and

ii) at least one inorganic UV filter which is doped by at least one transition metal

wherein the composite particles are spherical particles containing a core comprising at least one organic and/or one inorganic material, the said core being covered at least partially by at least one layer of inorganic UV filter which is doped by at least one transition metal.

The subject of the present invention concerns composite particles comprising:

i) a matrix containing at least one inorganic and/or at least one organic material and

ii) at least one inorganic UV filter which is doped by at least one transition metal

wherein the composite particles are spherical particles containing a core comprising at least one organic and/or one inorganic material, the said core being covered at least partially by at least one layer of inorganic UV filter which is doped by at least one transition metal.

The subject of the present invention is also a composition containing, in a physiologically acceptable medium, at least the composite particles as defined above.

The invention concerns also the composition as defined above for use in a cosmetic method for caring for and/or making up a keratinous substance, and more particularly in a cosmetic method for:

a) limiting the darkening of the skin and improving the colour and uniformity of the complexion, and
b) treating aging of keratinous substances.

**Description of Embodiments**

The following description and examples present other advantages, aspects and properties of the present invention.

**[Definitions]**

The following definitions are used in the present text.

The compositions according to the present invention are photoprotective compositions intended to screen out UV radiation; these compositions are also known as anti-sun compositions or sun protection compositions.

The expression "physiologically acceptable" means compatible with human keratinic materials in particular the skin and/or its appendages or mucous membranes, and having a pleasant colour, odour and feel and not causing any unacceptable discomfort (stinging, tautness or redness) liable to discourage the consumer from using this composition.

The expression "mean size of the particles" is understood to mean the parameter D[4,3] measured using a "Mastersizer 2000" particle size analyser (Malvern). The light intensity scattered by the particles as a function of the angle at which they are lit is converted to a size distribution according to Mie theory. The parameter D[4,3] is measured; this is the mean diameter of a sphere having the same volume as the particle. For a spherical particle, reference will often be made to the "mean diameter".

The expression "mean elementary size" means the size of non-aggregated particles.

The expression "human keratinic materials" means the skin (body, face, area around the eyes), scalp hair, eyelashes, eyebrows, body hair, nails, lips or mucous membranes.

The expression « inorganic UV filter" » means any compound which does not contain in its chemical structure any carbon atoms and which is able to filter radiation UV between 280 and 400 nm.

The expression "one inorganic UV filter (i.e., metal oxide as $TiO_2$, ZnO) which is doped by at least one transition metal" means any inorganic UV filter compound comprising a host lattice incorporating at least one transition metal to provide luminescence trap sites and/or killer sites. By luminescence trap sites and/or killer sites will be understood foreign ions designed to trap the electrons and positively charged holes and which therefore inhibit migration. The doped metal oxides and the methods for preparing them are described in WO9960994 and WO0140114.

**[Composite particles]**

The composite particles according to the invention are of spherical shape.

The term "spherical" is understood to mean that the particle has a sphericity index, i.e., the ratio between its largest diameter and its smallest diameter, of less than 1.2.

The composite particles according to the invention may further comprise composite particles of non-spherical shape.

The term "non-spherical" refers to particles in three dimensions (length, width and thickness or height) for which the ratio of the longest dimension to the shortest dimension is greater than 1.2. The dimensions of the particles of the invention are evaluated by scanning electron microscopy and image analysis. They comprise particles of parallelepipedal shape (rectangular or square surface), discoid shape (circular surface) or ellipsoid shape (oval surface), characterized by three dimensions: a length, a width and a height. When the shape is circular, the length and the width are identical and correspond to the diameter of a disc, whereas the height corresponds to the thickness of the disc. When the surface is oval, the length and the width correspond, respectively, to the large axis and the small axis of an ellipse, and the height corresponds to the thickness of the elliptic disc formed by the platelet. When it is a parallelepiped, the length and the width may be of identical or different dimensions: when they are of the same dimension, the shape of the surface of the parallelepiped is a square; in the contrary case, the shape is rectangular. As regards the height, it corresponds to the thickness of the parallelepiped.

The composite particles according to the invention may be mono- or multi-layered. The composite particles according to the invention preferably have a mean size of from 0.1 to 30 $\mu$m, preferably from 0.1 to 20 $\mu$m and even more preferentially from 0.1 to 10 $\mu$m.

According to a first variant, the composite particles contain a matrix comprising an organic and/or inorganic material, in which matrix particles of an inorganic UV-screening agent are included. According to this embodiment, the matrix has inclusions and particles of the inorganic UV-screening agent are placed in the inclusions of the matrix.

According to a second variant, the composite particles contain a matrix made of an organic and/or inorganic material, which matrix is covered at least partially with at least one layer of an inorganic UV-screening agent which may be connected to the matrix with the aid of a binder.

According to a third variant, the composite particles contain an inorganic UV-screening agent covered at least partially with at least one layer of an organic and/or inorganic material.

The matrix may also be formed from one or more organic or inorganic materials. It may then be a continuous phase of materials such as an alloy, i.e. a continuous phase in which the materials can no longer be dissociated, or a discontinuous phase of materials, for example constituted of an organic or inorganic material covered with a layer of another different organic or inorganic material.

According to one variant, in particular when the composite particles comprise a matrix covered with a layer of a UV-screening agent, the composite particles may further be covered with an additional coating, in particular chosen from biodegradable or biocompatible materials, lipid materials, for instance surfactants or emulsifiers, polymers, and oxides.

Preferably, the content of composite particles in the composition according to the invention ranges from 1% to 70%, preferably from 1.5% to 50% and more preferably from 2% to 40% by weight relative to the total weight of the composition.

**[Non-spherical composite particles]**

The organic materials that may be used to form the matrix of the non-spherical screening particles are chosen from the group formed by polyamides, silicones, polysaccharides, polyvinyl derivatives, waxes, and polyesters, and mixtures thereof.

Among the organic materials that may be used, mention is preferably made of:

- triethoxycaprylylsilane, and
- ethylene/vinyl acetate copolymers.

The inorganic materials that may be used in the matrix of the non-spherical composite particles are chosen from the group formed by mica, synthetic mica, talc, silica, aluminium oxide, boron nitride, kaolin, hydrotalcite, mineral clays, and synthetic clays, and mixtures thereof. Preferably, these inorganic materials are chosen from:

- silica,
- talc, and
- alumina.

The non-spherical composite particles of the invention are characterized by three dimensions, namely:

- the smallest is preferably greater than 0.1 μm, more preferably greater than 0.3 μm and better still greater than 0.5 μm, and
- the largest is preferably less than 30 μm, more preferably 20 μm and better still 10 μm.

The ratio of the largest to the smallest dimension is generally greater than 1.2.
The dimensions of the particles of the invention are evaluated by scanning electron microscopy and image analysis.
The non-spherical screening composite particles that may be used according to the invention will preferably be platelet-shaped.
The term "platelet-shaped" means parallelepipedal-shaped.
They may be smooth, rough or porous.
The platelet-shaped composite particles preferably have a mean thickness of from 0.1 to 10 μm, the mean length is generally from 0.5 to 30 μm and the mean width is from 0.5 to 30 μm.
The thickness is the smallest of the dimensions, the width is the medium dimension, and the length is the longest of the dimensions.
According to a first variant, the composite particles contain a matrix comprising an organic and/or inorganic material, in which matrix particles of an inorganic UV-screening agent are included.
According to a second variant, the composite particles contain a matrix made of an organic and/or inorganic material, covered at least partially with at least one layer of an inorganic UV-screening agent connected to the matrix by means of a binder.
According to this second variant, the mean thickness of the layer of inorganic UV-screening agent is generally about ten nanometres.
The mean thickness of the layer of inorganic UV-screening agent is advantageously from 0.001 to 0.2 μm and preferably from 0.01 to 0.15 μm.
According to a third variant, the non-spherical composite particles contain an inorganic UV-screening agent covered with at least one layer of an organic and/or inorganic material.
Preferably, the matrix of the composite particles contains a material or mixture of materials chosen from:

$SiO_2$,

alumina,

an alumina/triethoxycaprylylsilane mixture,

talc, and

Nylon.

More preferably, the matrix of the composite particles is formed from a material or mixture of materials chosen from:

alumina,

an alumina/triethoxycaprylylsilane mixture,

talc, and

silica.

**[Spherical composite particles]**

Acccording to the invention, the composite particles are spherical composite particles containing a core comprising at least one organic and/or one inorganic material, said core being covered at least partially by at least one layer of inorganic UV filter which is doped by at least one transition metal.
According to a first variant, the spherical composite particles contain a core comprising at least one organic and/or one inorganic material, in which matrix particles of inorganic UV-screening agent are included.

According to a second variant, the spherical composite particles contain a core comprising at least one organic and/or an inorganic material, covered at least partially with at least one layer of inorganic UV-screening agent which may be connected to the matrix with the aid of a binder.

According to this second variant, the mean thickness of the layer of inorganic UV-screening agent is generally from 0.001 to 0.2 μm and preferably from 0.01 to 0.15 μm.

According to a third variant, the spherical composite particles contain a core of an inorganic UV-screening agent covered at least partially with at least one layer of an organic and/or inorganic material.

The inorganic materials that may be used in the matrix of the spherical composite particles according to the present invention may be chosen from the group formed by glass, silica, and aluminium oxide, and mixtures thereof.

The organic materials that may be used to form the matrix are chosen from the group formed by cellulose, poly(meth)acrylates, polyamides, silicones, polyurethanes, polyethylenes, polypropylenes, polystyrenes, polycaprolactams, polysaccharides, polypeptides, polyvinyl derivatives, waxes, polyesters, and polyethers, and mixtures thereof.

The composite particles in spherical form are characterized by a mean diameter from 0.1 μm to 30 μm, preferably from 0.2 μm to 20 μm, more preferably from 0.3 μm to 10 μm, and advantageously from 0.5 μm to 10 μm.

The inorganic material or the organic material may be hollow or porous. The porosity of the material may be characterized by a specific surface area of from 0.05 $m^2/g$ to 1500 $m^2/g$, more preferentially from 0.1 $m^2/g$ to 1000 $m^2/g$ and even more preferentially from 0.2 $m^2/g$ to 500 m /g according to the BET method. Preferably, the matrix of the spherical composite particles contains a material or mixture of materials chosen from:

- $SiO_2$,
- cellulose
- polymethyl methacrylate,
- copolymers of styrene and of a $C_1/C_5$ alkyl (meth)acrylate derivative,
- polyamides, such as nylon.

Preferably, the matrix of the spherical composite particles contains as an organic material, porous or non porous spherical particles of crosslinked polymethyl methacrylate polymer like

- the product SSX-102® sold by the company SEKISUI PLASTICS,
- porous spherical particles of crosslinked polymethyl methacrylate polymer (mean size from 2 to 15 μm and specific area 75-95 $m^2/g$) such as the products sold under the commercial name TECHPOLYMER MBP-8® by the company SEKISUI PLASTICS, GANZ PEARL GMP-0800® by the company AICA KOGYO, and COVABEAD LH 85® by the company SENSIENT.

**[Preparation process]**

The composite particles according to the invention may be prepared by a mechanochemical fusion process.

A mechanochemical fusion process means a process in which mechanical power such as impact force, friction force or shear force is applied to a plurality of subjects to cause fusion between the subjects.

The mechanochemical fusion process may be performed by, for example, an apparatus comprising a rotating chamber and a fixed inner piece with a scraper, such as a mechanofusion system marketed by Hosokawa Micron Corporation in Japan.

It is preferable to use a hybridizer process as the mechanochemical fusion process.

The hybridizer process was developed in the 1980s. The hybridizer process is a class of mechanochemical fusion processes in which strong mechanical power is applied to a plurality of particles to cause a mechanochemical reaction to form a composite particle.

According to the hybridizer process, the mechanical power is imparted by a high speed rotor which can have a diameter from 10 cm to 1 m, and can rotate at a speed of 1,000 rpm to 100,000 rpm. Therefore, the hybridizer process can be defined as a mechanochemical fusion process using such a high speed rotor. The hybridizer process is performed in air or under dry conditions. Thus, due to the high speed rotation of the rotor, a high speed air flow may be generated near the rotor. However, some liquid materials may be subjected to the hybridizer process together with solid materials. The term "hybridizer process" has been used as a technical term.

The hybridizer process can be performed by using a hybridization system marketed by, for example, Nara Machinery® in Japan, in which at least two types of particles, typically core particles and fine particles, are fed into a

hybridizer equipped with a high speed rotor having a plurality of blades in a chamber under dry conditions, and the particles are dispersed in the chamber and mechanical and thermal energy (e.g., compression, friction and shear stress) are imparted to the particles for a relatively short period of time such as 1 to 10 minutes, preferably 1 to 5 minutes. As a result, one type of particles (e.g., fine particles) is embedded or fixed on the other type of particles (e.g., core particles) to form composite particles. It is preferable that the particles have been subjected to electrostatic treatment(s) such as shaking to form an "ordered mixture" in which one type of particles is spread to cover the other type of particles. The hybridizer process can also be performed by using a theta composer marketed by Tokuju Corporation® in Japan.

The hybridizer process can also be performed by using a Composi Hybrid® or a Mechano Hybrid® marketed by Nippon Coke.

**[Doped Inorganic UV Filter]**

The inorganic UV filter used according to the present invention is preferably composed of metal oxide particles and preferably selected from Zinc Oxide particles (ZnO), Titanium Dioxide particles ($TiO_2$), Iron-Oxide particles (FeO), and their mixtures.

Preferably the particles of $TiO_2$ (amorphous or crystallized in rutile and/or anatase form) are used. The rutile form is more preferred for its better photostability compared to the anatase form.

These metal oxides doped by at least one transition metal preferably have an elementary mean size from 0.001 to 0.2 $\mu$m, and advantageously, the metal oxide particles used have a mean elementary size from 0.01 to 0.15 $\mu$m.

The proportion of the metal doped inorganic UV filter is preferably from 5 to 80% by weight, more preferably from 5% to 60% by weight relative to the total weight of the composite particles.

These inorganic UV filter particles are doped by at least one transition metal preferably selected from Fe, Zn, Mn, Zr, Ce and their mixtures and more preferably selected from Fe and Mn. These metals can be incorporated singly or in mixtures. Further details of those doped metal oxides can be found in WO99/60994 and WO01/40114.

The optimum amount of the transition metal in the metal oxide may be determined by routine experimentation but it is preferably low enough so that the particles are not colored. Amounts as low as 0.1 mole% or above, for instance 5 mole% or 10 mole% can generally be used. Typical concentrations are from 0.5 to 2 mole% by weight relative to the total weight of the doped metal oxide.

The doped particles of metal oxide can be obtained by any one of the standard processes for preparing doped oxides. They can be obtained by a backing technique by combining particles of metal oxide with the transition metal in the form of a salt such as chloride or an oxygen-containing anion such as perchlorate or a nitrate, in solution or suspension, typically in solution in water, and then backing it, typically at a temperature of at least 300°C. Other routes which may be used to prepare the doped materials include a precipitation process of the type described in Mat. Sci. (1997) 36, 6001-6008 where solutions of the dopant salt and of an alkoxide of the host metal (i.e., Ti/Zn) are mixed, and the mixed solution is then heated to convert the alkoxide to the oxide. Heating is continued until a precipitate of the doped material is obtained. Further details can be found in WO99/60994 and WO01/40114.

According to a preferred embodiment, the doped metal oxide particles are selected from Fe doped $TiO_2$ particles and Mn doped $TiO_2$ particles.

As examples of metal oxide particles doped by at least one transition metal, mention may be made of the commercial products TTO F6 ® from Ishihara which is constituted of Fe doped titanium dioxide particles and OPTISOL OTP1-PW-WD® from Croda which is constituted of Mn doped titanium dioxide particles.

According to a particulary prefererred embodiment, the composite particles are spherical composite particles containing a core comprising at least a porous or non porous crosslinked polymethyl methacrylate polymer; said core being covered at least partially by at least one layer of Fe-doped $TiO_2$ particles.

According to another particulary prefererred embodiment, the composite particles are spherical composite particles containing a core comprising at least a porous or non porous crosslinked polymethyl methacrylate polymer; the said core being covered at least partially by at least one layer of Mn doped $TiO_2$ particles.

**[General Forms]**

The compositions according to the invention can be prepared according to techniques well known to those skilled in the art.

They may be in the form of an aqueous lotion or of an aqueous gel or comprise, in addition to the aqueous phase, at least one fatty phase and may be in the form of a simple or complex emulsion (O/W, W/O, O/W/O or W/O/W) such as a milk, of a cream or of a cream gel. They may optionally be packaged as an aerosol and may be in the form of a spray.

Preferably, the compositions according to the invention are in the form of an oil-in-water or water-in-oil emulsion, and more preferentially in water-in-oil form.

The emulsification processes that may be used are of paddle or impeller, rotor-stator and high-pressure homogenizer (HPH) type.

To obtain stable emulsions with a low content of emulsifying compounds (oil/emulsifier ratio > 25), it is possible to make the dispersion in concentrated phase and then to dilute the dispersion with the rest of the aqueous phase.

It is also possible, via HPH (between 50 and 800 bar), to obtain stable dispersions with drop sizes which can fall to 100 nm.

The emulsions generally comprise at least one emulsifier chosen from amphoteric, anionic, cationic or non-ionic emulsifiers, used alone or as a mixture. The emulsifiers are appropriately chosen according to the emulsion to be obtained (W/O or O/W). The emulsions may also contain stabilizers of other types, for instance fillers, or gelling or thickening polymers.

For the O/W emulsions, examples of emulsifiers that may be mentioned include non-ionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG-100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000 and Plantaren 1200, cetostearyl glucoside, optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of the mixture of arachidyl alcohol, behenyl alcohol and arachidyl glucoside, sold under the name Montanov 202 by the company SEPPIC. According to one particular embodiment of the invention, the mixture of the alkylpolyglucoside as defined above with the corresponding fatty alcohol can be in the form of a self-emulsifying composition, for example as described in document WO-A-92/06778.

Among the other emulsion stabilizers, use may also be made of isophthalic acid or sulfoisophthalic acid polymers, and in particular phthalate/sulfoisophthalate/glycol copolymers, for example the diethylene glycol/phthalate/isophthalate/1,4-cyclohexanedimethanol copolymer (INCI name: Polyester-5) sold under the names Eastman AQ Polymer (AQ35S, AQ38S, AQ55S and AQ48 Ultra) by the company Eastman Chemical.

Among the other emulsion stabilizers, mention may also be made of hydrophobically modified 2-acrylamido-2-methylpropanesulfonic acid polymers such as those described in patent application EP 1 069 142.

When it is an emulsion, the aqueous phase of the latter can comprise a non-ionic vesicular dispersion prepared according to known processes (Bangham, Standish and Watkins, J. Mol. Biol., 13, 238 (1965), FR 2 315 991 and FR 2 416 008).

As emulsifying surfactants that can be used for the preparation of W/O emulsions, mention may be made, for example, of emulsifying surfactants having an HLB of less than or equal to 5 at 25°C.

The term HLB (hydrophilic lipophilic balance) is well known to those skilled in the art, and denotes the hydrophilic-lipophilic balance of a surfactant.

The HLB of the surfactant(s) used according to the invention is the HLB according to Griffin, defined in the publication J. Soc. Cosm. Chem. 1954 (volume 5), pages 249-256.

Non-limiting examples of surfactants with an HLB of less than or equal to 5 are especially given in the publication entitled McCutcheon's Emulsifiers & Detergents, 1998 International Edition, MC Publishing Company, in the chapter entitled HLB Index.

Examples of W/O emulsifying surfactants that may be mentioned include alkyl esters or ethers of sorbitan, of glycerol, of polyol or of sugars; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE 09 by the company Goldschmidt. One or more coemulsifiers may also be added thereto, which may be chosen advantageously from the group consisting of polyol alkyl esters.

Non-silicone emulsifiers, in particular alkyl esters or ethers of sorbitan, of glycerol, of polyol or of sugars, are preferred.

Alkyl esters of polyol that may in particular be mentioned include polyethylene glycol esters, for instance PEG-30 dipolyhydroxystearate, such as the product sold under the name Arlacel P135 by the company ICI.

Esters of glycerol and/or of sorbitan that may be mentioned include, for example, polyglyceryl isostearate, such as the product sold under the name Isolan GI 34 by the company Goldschmidt, sorbitan isostearate, such as the product sold under the name Arlacel 987 by the company ICI, sorbitan glyceryl isostearate, such as the product sold under the name Arlacel 986 by the company ICI, and mixtures thereof.

According to one particularly preferred form of the invention, the compositions are in the form of a water-in-oil

emulsion.

**[Aqueous Phase]**

When the compositions of the invention contain an aqueous phase, said phase contains water and optionally other water-soluble or water-miscible organic solvents.

An aqueous phase suitable for the invention can comprise, for example, water chosen from natural spring water, such as water from La Roche-Posay, water from Vittel or waters from Vichy, or floral water.

The water-soluble or water-miscible solvents suitable for the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol, and sorbitol, and mixtures thereof.

According to a preferred embodiment, use may more particularly be made of ethanol, propylene glycol, glycerol, and mixtures thereof.

The water or the aqueous phase (i.e., in a emulsion) may be present in a composition of the invention in a content ranging from 5% to 90% by weight, in particular from 5% to 75% by weight and more particularly from 10% to 70% by weight, relative to the total weight of said composition.

A water-soluble organic solvent may be present in a composition of the invention in a content ranging from 1% to 30% by weight and in particular from 2% to 20% by weight, relative to the total weight of said composition.

**[Oily Phase]**

The composition of the invention may also comprise at least one oily phase comprising at least one oil.

For the purposes of the invention, the term "oily phase" is understood to mean a phase comprising at least one oil and all of the liposoluble and lipophilic ingredients and the fatty substances used for the formulation of the compositions of the invention.

The term "oil" is understood to mean any fatty substance in liquid form at ambient temperature (20 - 25°C) and at atmospheric pressure (760 mmHg).

An oil suitable for the invention can be volatile or non-volatile.

An oil suitable for the invention can be chosen from hydrocarbon-based oils, silicone oils, fluorinated oils and mixtures thereof.

A hydrocarbon-based oil suitable for the invention can be an animal hydrocarbon-based oil, a vegetable hydrocarbon-based oil, a mineral hydrocarbon-based oil or a synthetic hydrocarbon-based oil.

An oil suitable for the invention can advantageously be chosen from mineral hydrocarbon-based oils, vegetable hydrocarbon-based oils, synthetic hydrocarbon-based oils, silicone oils and mixtures thereof.

For the purposes of the present invention, the term "silicone oil" is understood to mean an oil comprising at least one silicon atom, and in particular at least one Si-O group.

The term "hydrocarbon-based oil" is understood to mean an oil comprising mainly hydrogen and carbon atoms.

For the purposes of the present invention, the term "silicone oil" is understood to mean an oil comprising at least one silicon atom, and in particular at least one Si-O group.

The term "fluorinated oil" is understood to mean an oil comprising at least one fluorine atom.

A hydrocarbon-based oil suitable for the invention can in addition optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent or agents, at least one volatile or non-volatile hydrocarbon-based oil and/or one volatile and/or non-volatile silicone oil.

For the purposes of the invention, the term "volatile oil" is understood to mean an oil capable of evaporating on contact with the skin or the keratin fibre in less than one hour, at ambient temperature and atmospheric pressure. The volatile oil(s) of the invention are volatile cosmetic oils which are liquid at ambient temperature and which have a non-zero vapour pressure, at ambient temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at ambient temperature and atmospheric pressure for at least several hours, and that especially has a vapour pressure of less than $10^{-3}$ mmHg (0.13 Pa).

(Hydrocarbon-based oils)

Mention may in particular be made, as non-volatile hydrocarbon-based oils which can be used according to the invention, of:

(i) hydrocarbon-based oils of vegetable origin such as triglyceride esters, which are generally fatty acid triesters of glycerol, the fatty acids of which may have chain lengths varying from $C_4$ to $C_{24}$, these chains possibly being linear or branched, and saturated or unsaturated; these oils are especially wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; or else caprylic/capric acid triglycerides, for instance those sold by the company Stéarineries Dubois or those sold under the names Miglyol 810, 812 and 818 by the company Dynamit Nobel;

(ii) synthetic ethers containing from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, such as the oils of formula RCOOR' in which R represents the residue of a linear or branched fatty acid comprising from 1 to 40 carbon atoms and R' represents a hydrocarbon-based chain, in particular a branched hydrocarbon-based chain, comprising from 1 to 40 carbon atoms, provided that R + R' is ≥10, such as, for example, purcellin oil (cetearyl octanoate), isopropyl myristate, isopropyl palmitate, $C_{12}$-$C_{15}$ alkyl benzoate, such as the product sold under the trade name Finsolv TN or Witconol TN by Witco or Tegosoft TN by Evonik Goldschmidt, 2-ethylphenyl benzoate, such as the commercial product sold under the name X-Tend 226 by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI by Enichem Augusta Industriale, and also di(linear $C_{14}$-$C_{15}$ alkyl) tartrates, such as those sold under the name Cosmacol ETL by the same company; or acetates;

(v) fatty alcohols which are liquid at ambient temperature, comprising a branched and/or unsaturated carbon chain having from 12 to 26 carbon atoms, such as octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates such as dicaprylyl carbonate, for instance the product sold under the name Cetiol CC by the company Cognis;

(viii) fatty amides, such as isopropyl N-lauroyl sarcosinate, such as the product sold under the trade name Eldew SL205 from Ajinomoto;

and mixtures thereof.

Mention may in particular be made, as volatile hydrocarbon-based oils which can be used according to the invention, of hydrocarbon-based oils having from 8 to 16 carbon atoms, in particular branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, or the alkanes described in the patent applications from Cognis, WO 2007/068371 or WO 2008/155059 (mixtures of different alkanes differing by at least one carbon). These alkanes are obtained from fatty alcohols, which are themselves obtained from coconut oil or palm oil, the oils sold under the trade name Isopar or Permethyl, branched $C_8$-$C_{16}$ esters, isohexyl neopentanoate, and mixtures thereof.

Other volatile hydrocarbon-based oils, for instance petroleum distillates, especially those sold under the name Shell Solt by the company Shell, may also be used. According to one embodiment, the volatile solvent is chosen from volatile hydrocarbon-based oils having from 8 to 16 carbon atoms, and mixtures thereof.

(Silicone oils)

The non-volatile silicone oils can be chosen in particular from non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Mention may be made, as volatile silicone oils, for example, of volatile linear or cyclic silicone oils, in particular those having a viscosity ≤ 8 centistokes ($8x10^{-6}$ $m^2/s$) and having in particular from 2 to 7 silicon atoms, these silicones optionally comprising alkyl or alkoxy groups having from 1 to 10 carbon atoms. Mention may in particular be made, as volatile silicone oils which can be used in the invention, of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane, and mixtures thereof.

Mention may also be made of the volatile linear alkyltrisiloxane oils of general formula (I):

[formula (I)]

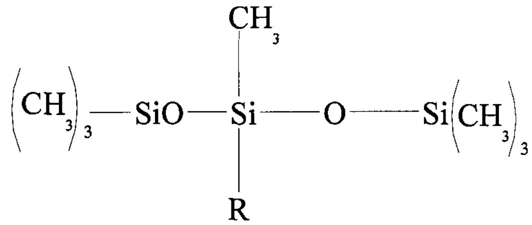

where R represents an alkyl group comprising from 2 to 4 carbon atoms, one or more hydrogen atoms of which can be replaced by a fluorine or chlorine atom.

Mention may be made, among the oils of general formula (I), of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

(Fluorinated oils)

Use may also be made of volatile fluorinated oils, such as nonafluoromethoxybutane, decafluoropentane, tetradecafluorohexane, dodecafluoropentane, and mixtures thereof.

The oily phase (excluding a lipophilic organic UV-screening agent) preferably ranges from 3% to 60% by weight and preferably from 5% to 30% by weight relative to the total weight of the composition.

An oily phase according to the invention can additionally comprise other fatty substances, mixed with or dissolved in the oil.

Another fatty substance which can be present in the oily phase can be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

**[Additives]**

The compositions in accordance with the present invention may also comprise standard cosmetic adjuvants chosen in particular from organic solvents, ionic or non-ionic, hydrophilic or lipophilic thickeners, demulcents, humectants,

opacifiers, stabilizers, emollients, silicones, antifoams, fragrances, preserving agents, anionic, cationic, non-ionic, zwitterionic or amphoteric surfactants, fillers, polymers, propellants, basifying or acidifying agents or any other ingredient commonly used in the cosmetics and/or dermatological fields.

Among the organic solvents that may be mentioned are lower alcohols and polyols. The latter can be chosen from glycols and glycol ethers, such as ethylene glycol, propylene glycol, butylene glycol, dipropylene glycol or diethylene glycol.

Hydrophilic thickeners that may be mentioned include carboxyvinyl polymers such as the Carbopol products (carbomers) and the Pemulen products (acrylate/$C_{10}$-$C_{30}$-alkyl acrylate copolymer); polyacrylamides, for instance the crosslinked copolymers sold under the names Sepigel 305 (CTFA name: polyacrylamide/$C_{13}$-$C_{14}$ isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Clariant under the trade name Hostacerin AMPS (CTFA name: ammonium polyacryloyldimethyl taurate) or Simulgel 800 sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose; polysaccharides and especially gums such as xanthan gum; and mixtures thereof.

Lipophilic thickeners that may be mentioned include synthetic polymers such as poly($C_{10}$-$C_{30}$ alkyl acrylates) sold under the name Intelimer IPA 13-1 and Intelimer IPA 13-6 by the company Landec, or modified clays such as hectorite and its derivatives, for instance the products sold under the name Bentone.

The compositions according to the invention may also furthermore comprise additional cosmetic and dermatological active agents.

Mention may be made, among active agents, of:

- vitamins (A, C, E, K, PP, and the like) and their derivatives or precursors, alone or as mixtures;
- antioxidants;
- free-radical scavengers;
- antiglycation agents;
- soothing agents;
- NO-synthase inhibitors;
- agents which stimulate the synthesis of dermal or epidermal macromolecules and/or which prevent their decomposition;
- agents which stimulate the proliferation of fibroblasts;
- agents which stimulate the proliferation of keratinocytes;
- muscle relaxants;
- tensioning agents;
- mattifying agents;
- keratolytic agents;
- desquamating agents;
- moisturizers, for instance polyols such as glycerol, butylene glycol or propylene glycol;
- anti-inflammatory agents;
- agents which act on the energy metabolism of cells;
- insect repellents;
- substance P antagonists or CGRP antagonists;
- agents for combating hair loss and/or for restoring the hair;
- anti-wrinkle agents;
- additional inorganic and organic UV-screening agents such as those previously mentioned.

According to the invention, the compositions may further contain one or more complementary hydrophilic, lipophilic or insoluble organic screening agents and/or one or more inorganic screening agents under a free form which are active in UVA and/or UVB radiations.

The complementary organic screening agents are chosen more preferably from dibenzoylmethane compounds; anthranilates; cinnamic compounds; salicylic compounds; camphor compounds; benzophenone compounds; β,β-diphenylacrylate compounds; triazine derivatives; benzalmalonate compounds, especially those mentioned in the patent US 5 624 663; benzimidazole compounds; imidazolines; p-aminobenzoic acid (PABA) compounds; benzotriazole compounds; methylenebis(hydroxyphenylbenzotriazole) compounds as described in the following patent applications US 5 237 071, US 5 166 355, GB 2 303 549, DE 197 26 184 and EP 893 119; benzoxazole compounds as described in the following patent applications EP 0 832 642, EP 1 027 883, EP 1 300 137 and DE 101 62 844; screening polymers and

screening silicones such as those described especially in the patent application WO 93/04665; α-alkylstyrene-based dimers, such as those described in the patent application DE 198 55 649; 4,4-diarylbutadienes such as those described in the following patent applications EP 0 967 200, DE 197 46 654, DE 197 55 649, EP-A-1 008 586, EP 1 133 980 and EP 133 981; merocyanine derivatives such as those described in the following patent applications WO 04/006878, WO 05/058269 and WO 06/032741; the indanylidene screening agents of the patents EP-A-0 823 418 and EP-A-1 341 752 and their mixtures.

As examples of complementary organic UV-screening agents, mention may be made of those denoted hereinbelow under their INCI name:

Dibenzoylmethane derivatives:
Butylmethoxydibenzoylmethane, sold under the trade name Parsol 1789® by the company DSM Nutritional Products.

Para-Aminobenzoic acid derivatives:

PABA,
Ethyl PABA,
Ethyl dihydroxypropyl PABA,
Ethylhexyl dimethyl PABA sold in particular under the name Escalol 507 by ISP, Glyceryl PABA,
PEG-25 PABA sold under the name Uvinul P25 by BASF,

Salicylic derivatives:

Homosalate sold under the name Eusolex HMS® by Merck,
Ethylhexyl salicylate sold under the name Neo Heliopan OS® by Symrise,
Dipropylene glycol salicylate sold under the name Dipsal® by Lubrizol,
TEA salicylate sold under the name Neo Heliopan TS® by Symrise.

Cinnamic derivatives:

Ethylhexyl methoxycinnamate sold especially under the trade name Parsol MCX ® by DSM Nutritional Products,
Isopropyl methoxycinnamate,
Isoamyl methoxycinnamate sold under the trade name Neo Heliopan E 1000® by Symrise,
Cinoxate,
DEA Methoxycinnamate,
Diisopropyl methylcinnamate,
Glyceryl ethylhexanoate dimethoxycinnamate.

β, β-Diphenylacrylate derivatives:

Octocrylene sold especially under the trade name Uvinul N539® by BASF,
Etocrylene sold especially under the trade name Uvinul N35® by BASF.

Benzophenone derivatives:

Benzophenone-1 sold under the trade name Uvinul 400® by BASF,
Benzophenone-2 sold under the trade name Uvinul D50® by BASF,
Benzophenone-3 or Oxybenzone sold under the trade name Uvinul M40 by BASF,
Benzophenone-4 sold under the trade name Uvinul MS40® by BASF,
Benzophenone-5,
Benzophenone-6 sold under the trade name Helisorb 11® by Norquay,
Benzophenone-8 sold under the trade name Cyasorb UV-24® by Cytec,
Benzophenone-9 sold under the trade name Uvinul DS-49® by BASF,
Benzophenone-12,
n-hexyl 2-(4-diethylamino-2-hydroxybenzoyl)benzoate sold under the trade name Uvinul A PLUS ® or in the form of a mixture with octyl methoxycinnamate under the trade name Uvinul A PLUS B by BASF,
1,1'-(1,4-piperazinediyl)bis[1-[2-[4-(diethylamino)-2-hydroxybenzoyl]phenyl]methanone (CAS 919803-06-8).

Benzylidene camphor derivatives:

3-Benzylidenecamphor manufactured under the name Mexoryl SD® by Chimex,
4-Methylbenzylidenecamphor sold under the name Eusolex 6300® by Merck,
Benzylidenecamphorsulfonic acid manufactured under the name Mexoryl SL® by Chimex,
Camphor benzalkonium methosulfate manufactured under the name Mexoryl SO® by Chimex,
Terephthalylidenedicamphorsulfonic acid manufactured under the name Mexoryl SX® by Chimex,
Polyacrylamidomethylbenzylidenecamphor manufactured under the name Mexoryl SW® by Chimex.

Phenylbenzimidazole derivatives:

Phenylbenzimidazolesulfonic acid sold in particular under the trade name Eusolex 232® by Merck,
Disodium phenyl dibenzimidazole tetrasulfonate sold under the trade name Neo Heliopan AP® by Symrise.

Benzotriazole derivatives:

Drometrizole trisiloxane sold under the name Silatrizole by Rhodia Chimie,
Methylenebis(benzotriazolyl)tetramethylbutylphenol sold in solid form under the trade name MIXXIM BB/100® by Fairmount Chemical, or in micronized form as an aqueous dispersion under the trade name Tinosorb M® by BASF.

Triazine derivatives:

Bis(ethylhexyloxyphenol)methoxyphenyltriazine sold under the trade name Tinosorb S® by BASF,
Ethylhexyltriazone sold in particular under the trade name Uvinul T150® by BASF,
Diethylhexylbutamidotriazone sold under the trade name Uvasorb HEB® by Sigma 3V,
The silicone triazine substituted by two aminobenzoate groups such as those disclosed in the patent EP0841341 in particular the compound 2,4-Bis(n-butyl 4'-aminobenzalmalonate)-6-[(3-{1,3,3,3-tetramethyl-1-[(trimethylsilyloxy]-disiloxanyl}propyl)amino]-s-triazine,
2,4,6-Tris(diisobutyl 4'-aminobenzalmalonate)-s-triazine,
2,4,6-Tris(dineopentyl 4'-aminobenzalmalonate)-s-triazine,
2,4-Bis(dineopentyl 4'-aminobenzalmalonate)-6-(n-butyl 4'-aminobenzoate)-s-triazine,
the symmetrical triazine screening agents described in the patent US 6 225 467,
the patent application WO 2004/085412 (see compounds 6 and 9) or the document "Symmetrical Triazine Derivatives" IP.COM Journal, IP.COM INC West Henrietta, NY, US (20 September 2004), especially 2,4,6-tris(biphenyl)-1,3,5-triazines (in particular 2,4,6-tris(biphenyl-4-yl-1,3,5-triazine) and 2,4,6-tris(terphenyl)-1,3,5-triazine which is also mentioned in the Beiersdorf patent applications WO 06/035000, WO 06/034982, WO 06/034991, WO 06/035007, WO 2006/034992 and WO 2006/034985.

Anthranilic derivatives:
Menthyl anthranilate sold under the trade name Neo Heliopan MA® by Symrise.

Imidazoline derivatives:
Ethylhexyl dimethoxybenzylidene dioxoimidazoline propionate.

Benzalmalonate derivatives:

Dineopentyl 4'-methoxybenzalmalonate,
Polyorganosiloxane containing benzalmalonate functions, for instance Polysilicone-15, sold under the trade name Parsol SLX® by DSM Nutritional Products.

4,4-Diarylbutadiene derivatives:
1,1-Dicarboxy(2,2'-dimethylpropyl)-4,4-diphenylbutadiene.

Benzoxazole derivatives:

2,4-Bis[5-1(dimethylpropyl)benzoxazol-2-yl-(4-phenyl)imino]-6-(2-ethylhexyl)imino-1,3,5-triazine,
and mixtures thereof.

The complementary organic UV-screening agents are preferably present in the compositions according to the

invention in proportions ranging from 0.01% to 20% by weight relative to the total weight of the composition, and preferably ranging from 0.1% to 15% by weight relative to the total weight of the composition.

The complementary inorganic screening agents under a free form are chosen from coated or uncoated metal oxide pigments in which the mean size of the primary particles is preferentially between 5 nm and 100 nm (preferably between 10 nm and 50 nm), for instance titanium oxide (amorphous or crystallized in rutile and/or anatase form), iron oxide, zinc oxide, zirconium oxide or cerium oxide pigments, which are all UV-photoprotective agents that are well known per se.

The pigments may be coated or uncoated.

The coated pigments are pigments that have undergone one or more surface treatments of chemical, electronic, mechanochemical and/or mechanical nature with compounds as described, for example, in Cosmetics & Toiletries, February 1990, Vol. 105, pp. 53-64, such as amino acids, beeswax, fatty acids, fatty alcohols, anionic surfactants, lecithins, sodium, potassium, zinc, iron or aluminium salts of fatty acids, metal alkoxides (titanium or aluminium alkoxides), polyethylene, silicones, proteins (collagen, elastin), alkanolamines, silicon oxides, metal oxides or sodium hexametaphosphate.

As is known, silicones are organosilicon polymers or oligomers of linear or cyclic, branched or crosslinked structure, of variable molecular weight, obtained by polymerization and/or polycondensation of suitably functionalized silanes, and consist essentially of a repetition of main units in which the silicon atoms are linked together via oxygen atoms (siloxane bond), optionally substituted hydrocarbon-based radicals being directly attached via a carbon atom to the said silicon atoms.

The term "silicones" also includes the silanes required for their preparation, in particular alkylsilanes.

The silicones used for the coating of the pigments suitable for the present invention are preferably chosen from the group consisting of alkylsilanes, polydialkylsiloxanes and polyalkylhydrosiloxanes. More preferably still, the silicones are chosen from the group containing octyltrimethylsilane, polydimethylsiloxanes and polymethylhydrosiloxanes.

Of course, before being treated with silicones, the metal oxide pigments may have been treated with other surface agents, in particular with cerium oxide, alumina, silica, aluminium compounds or silicon compounds, or mixtures thereof.

Such metal oxide pigments, coated or non coated are in particular disclosed in the patent application EP-A-0 518 773. As commercial pigments, mention may be made of the products sold by the companies Sachtleben, Tayca, Merck, and Evonik.

The complementary inorganic UV filters according represents generally from 0.5 to 40%, preferably from 1 to 30%, by weight relative to the total weight of the composition.

Needless to say, those skilled in the art will take care to select the optional additional compound(s) mentioned above and/or the amounts thereof such that the advantageous properties intrinsically associated with the compositions in accordance with the invention are not, or are not substantially, adversely affected by the envisaged addition(s).

Those skilled in the art will choose said active agent(s) according to the desired effect on the skin, hair, eyelashes, eyebrows or nails.

The cosmetic compositions according to the invention have applications in a great number of treatments, in particular cosmetic treatments, of the skin, lips and hair, including the scalp.

Another subject of the present invention is the use of the compositions according to the invention as defined above in the manufacture of products for the cosmetic treatment of the skin, lips, nails, hair, eyelashes, eyebrows and/or scalp, in particular care products, anti-sun products and make-up products.

The cosmetic compositions according to the invention can be used, for example, as make-up products.

The cosmetic compositions according to the invention may be used, for example, as care products and/or anti-sun protection products for the face and/or the body, of liquid to semi-liquid consistency, such as milks, more or less rich creams, cream-gels and pastes. They may optionally be packaged in aerosol form and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged as an aerosol in accordance with the invention generally comprise conventional propellants, such as, for example, hydrofluorinated compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

The examples that follow serve to illustrate the invention.

## Examples

**Examples 1 to 4:** Preparation of composite particles by means of a mechanofusion hybridization process

The constituents indicated in Table 1 were subjected to hybridization using a hybridizer equipped with a high-speed rotor having a plurality of blades in a chamber under dry conditions, sold by Nara Machinery Co., Ltd in Japan. For each Example, the constituents indicated in Table 1 were mixed, in the weight ratio indicated in the same table, in a plastic bag which was shaken for a few minutes. The mixture was then placed in the hybridizer and the rotor was rotated at 8000 rpm (linear speed of 100 m/s) for 3 minutes.

Table 1

| Composite Particles | Matrix | Inorganic UV Filter |
|---|---|---|
| **Ex. 1 (invention)** | PMMA (1) 90% by weight | Mn-Doped-$TiO_2$ (4) 10% by weight |
| **Ex. 2 (invention)** | PMMA (2) 80% by weight | Fe-Doped $TiO_2$ (5) 20% by weight |
| **Ex.3 (comparative)** | Nylon (3) 70% by weight | Non-Doped $TiO_2$ (6) 30% by weight |
| **Ex. 4 (comparative)** | PMMA (2) 80% by weight | Non-Doped $TiO_2$ (6) 20% by weight |
| (1) SSX-102® sold by SEKISUI PLASTICS<br>(2) COVABEAD LH85® sold by SENSIENT<br>(3) TORAY SP-500®<br>(4) OPTISOL OTP1-PW-WD® by CRODA<br>(5) TTO F6 ® by Ishihara which is constituted of Fe-doped titanium dioxide<br>(6) MT-100 TV sold by Tayca | | |

**Examples 5 to 9: anti-sun water/oil emulsions**

Compositions 5 to 9 below were prepared. The ingredients are given as weight percentages of active material relative to the total weight of the composition.

Table 2

| Phase | Ingredients | Ex. 5 | Ex. 6 | Ex. 7 | Ex. 8 | Ex. 9 |
|---|---|---|---|---|---|---|
| A1 | Water | 42.00 | 42.00 | 42.00 | 42.00 | 42.00 |
| | Preservative | 0.40 | 0.40 | 0.40 | 0.40 | 0.40 |
| | Glycerin | 6.00 | 6.00 | 6.00 | 6.00 | 6.00 |
| | Glycol | 6.00 | 6.00 | 6.00 | 6.00 | 6.00 |
| | Terephthalylidene Dicamphor Sulfonic Acid (MEXORYL SX®) | 0.90 | 0.90 | 0.90 | 0.90 | 0.90 |
| | Triethanolamine | 0.16 | 0.16 | 0.16 | 0.16 | 0.16 |
| A2 | EDTA | 0.20 | 0.20 | 0.20 | 0.20 | 0.20 |
| A3 | Potassium Cetyl Phosphate (AMPHISOL K®) | 1.00 | 1.00 | 1.00 | 1.00 | 1.00 |
| B1 | $C_{12}$-$C_{15}$ Alkyl Benzoate (FINSOLV TN®) | 7.50 | 7.50 | 7.50 | 7.50 | 7.50 |
| | Octocrylene (Uvinul N539 T®) | 7.00 | 7.00 | 7.00 | 7.00 | 7.00 |
| | Avobenzone (PARSOL 1789®) | 3.00 | 3.00 | 3.00 | 3.00 | 3.00 |
| | Ethylhexyl Triazone (UVINUL T150®) | 0.50 | 0.50 | 0.50 | 0.50 | 0.50 |

(continued)

| Phase | Ingredients | Ex. 5 | Ex. 6 | Ex. 7 | Ex. 8 | Ex. 9 |
|---|---|---|---|---|---|---|
| B2 | Stearic acid | 1.00 | 1.00 | 1.00 | 1.00 | 1.00 |
| | Triethanolamine | 0.30 | 0,30 | 0.30 | 0.30 | 0.30 |
| | GLYCERYL STEARATE (and) PEG-100 STEARATE (Arlacel 165 FL®) | 1.00 | 1.00 | 1.00 | 1.00 | 1.00 |
| | Dimethicone | 0.50 | 0.50 | 0.50 | 0.50 | 0.50 |
| | Preservative | 1.00 | 1.00 | 1.00 | 1.00 | 1.00 |
| B3 | Mn-doped titanium dioxide (OPTISOL OTP1-PW-WD®) | - | 0.99 | - | - | - |
| C | Isohexadecane | 4.50 | 4.50 | 4.50 | 4.50 | 4.50 |
| | ACRYLATES/ $C_{10-30}$ ALKYL ACRYLATE CROSS POLY-MER (Pemulen TR-1®) | 0.25 | 0.25 | 0.25 | 0.25 | 0.25 |
| | Xanthane Gum | 0.10 | 0.10 | 0.10 | 0.10 | 0.10 |
| D | Water | 1.00 | 1.00 | 1.00 | 1.00 | 1.00 |
| | Triethanolamine | 0.25 | 0.25 | 0.25 | 0.25 | 0.25 |
| E | Particles of Example 1 | 5.00 | - | - | | |
| | Particles of Example 2 | | - | - | 2.5 | - |
| | Particles of Example 3 | - | - | 1.67 | - | - |
| | Particles of Example 4 | - | - | - | - | 2.5 |
| F | Water | qsp 100 | qsp 100 | qsp 100 | qsp 100 | qsp 100 |
| G | Alcohol | 2.00 | 2.00 | 2.00 | 2.00 | 2.00 |

**Emulsion preparation method:**

The aqueous phase A1, A2, A3 and oily phase B1 and B2 are prepared by mixing the raw materials, with mechanical stirring, at 80°C; the solutions obtained are macroscopically homogeneous. The emulsion is prepared by slow introduction of the oily phase into the aqueous phase with stirring using a Moritz homogenizer at a stirring speed of 4,000 rpm for 15 minutes. The emulsion obtained is cooled, with stirring, to 40°C, then the phase B3 is added thereto with gentle stirring, followed by phases C, D, E, and F. The emulsion obtained is cooled to room temperature with gentle stirring and then phase G added. It is characterized by drops between 1 μm and 10 μm in size.

These compositions were evaluated according to the following properties:

- screening efficacy, and
- sensoriality on application to the skin.

Examples 5 to 9 were compared with an equal active material amount of $TiO_2$ at 0.5% by weight:

(*In vitro* SPF)

The sun protection factor (SPF) is determined according to the *in vitro* method described by B.L. Diffey in J. Soc. Cosmet. Chem. 40, 127-133 (1989). The measurements were carried out using a UV-1000S spectrophotometer from the company Labsphere. Each composition is applied to a rough plate of PMMA in the form of a uniform and even deposit in a proportion of 1 mg/cm$^2$.

(*In vitro* PPD index)

To measure the UV-A protection index, the PPD (persistent pigment darkening) method, which measures the skin colour observed 2 to 4 hours after exposing the skin to UV-A, is particularly recommended and used. This method

has been adopted since 1996 by the Japanese Cosmetic Industry Association (JCIA) as the official test procedure for the UV-A labelling of products and is frequently used by test laboratories in Europe and the United States (Japan Cosmetic Industry Association Technical Bulletin, Measurement Standards for UVA protection efficacy, Issued November 21, 1995 and effective as of January 1, 1996).

The $UVA_{PPD}$ sun protection factor ($UVA_{PPD}$ PF) is expressed mathematically by the ratio of the UV-A radiation dose necessary to reach the pigmentation threshold with the UV-screening agent (MPPDp) to the UV-A radiation dose necessary to reach the pigmentation threshold without UV-screening agent (MPPDnp).

[Math. 1]

$$UVA_{PPD}PF = \frac{MPPDp}{MPPDnp}$$

The measurements were carried out according to an *in vitro* test using a UV-2000S spectrophotometer from the company Labsphere. Each composition is applied to a rough plate of PMMA in the form of a uniform and even deposit in a proportion of 1 $mg/cm^2$.

(Protocol for evaluating the sensory effect after application to the skin)

The sensory effect after application of the formula to the skin is evaluated by applying the formula to a forearm at a rate of 2 $mg/cm^2$, waiting for a drying time equal to 2 minutes and then assessing the friction force felt between the fingers and the surface of the forearm. The greasy oil skin sensation was then evaluated after application.

(Results)

Table 3

| Examples | *In vitro* SPF | *In vitro* PPD | *Sensory effect after application* |
|---|---|---|---|
| **Example 5 (invention)** | 37.19 +/- 1.04 | 20.87 +/- 0.54 | + |
| **Example 6 (comparative)** | 32.04 +/- 4.07 | 17.95 +/- 2.33 | - |
| **Example 7 (comparative)** | 29.11 +/-0.1 | 16.71 +/- 0.96 | - |
| **Example 8 (invention)** | 33.7 +/-1.71 | 19.38 +/- 1.2 | + |
| **Example 9 (comparative)** | 24.48 +/- 1.93 | 14.82 +/- 1.53 | - |
| + means non oily and non greasy sensation after application<br>- means oily and greasy sensation after application | | | |

These results show that

- compositions 5 and 8 according to the invention containing composite particles with doped $TiO_2$ are more efficient against UVA and UVB radiation than comparative compositions 7 and 9 containing composite particles with non-doped $TiO_2$.
- compositions 5 and 8 according to the invention containing composite particles with doped $TiO_2$ have better sensorial effect than comparative composition 6 containing doped $TiO_2$ particles in a free form and comparative compositions 7 and 9 containing composite particles with non-doped $TiO_2$.

## Claims

**1.** Composite particles comprising:

i) a matrix containing at least one inorganic and/or at least one organic material and
ii) at least one inorganic UV filter which is doped by at least one transition metal, wherein the composite particles are spherical particles containing a core comprising at least one organic and/or one inorganic material, the said core being covered at least partially by at least one layer of inorganic UV filter which is doped by at least one transition metal.

**2.** Composite particles according to claim 1, which have a mean size of from 0.1 to 30 μm, preferably from 0.1 to 20 μm and more preferentially from 0.1 to 10 μm.

**3.** Composite particles according to claim 1 or 2, **characterized in that**

- the inorganic material is chosen from the group formed by glass, silica, and aluminium oxide, and mixtures thereof,
- the organic material is chosen from the group formed by cellulose, poly(meth)acrylates, polyamides, silicones, polyurethanes, polyethylenes, polypropylenes, polystyrenes, polycaprolactams, polysaccharides, polypeptides, polyvinyls, waxes, polyesters, and polyethers, and mixtures thereof.

**4.** Composite particles according to any one of claims 1 to 3, **characterized in that** the core contains porous or non-porous spherical particles of a crosslinked polymethyl methacrylate polymer.

**5.** Composite particles according to any one of claims 1 to 4, wherein particles of the inorganic UV filter are composed of metal oxide particles and preferably selected from Zinc Oxide particles (ZnO), Titanium Dioxide particles ($TiO_2$), Iron Oxide particles (FeO), and their mixtures and more preferably particles of $TiO_2$.

**6.** Composite particles according to any one of claims 1 to 5, wherein particles of the inorganic UV filter have an elementary mean size from 0.001 to 0.2 μm, and advantageously, have a mean elementary size from 0.01 to 0.15 μm.

**7.** Composite particles according to any one of claims 1 to 6, wherein the proportion of the metal-doped inorganic UV filter is from 5 to 80% by weight, more preferably from 5% to 60% by weight relative to the total weight of the composite particles.

**8.** Composite particles according to any one of claims 1 to 7, wherein particles of the inorganic UV filter are doped by at least one transition metal selected from Fe, Zn, Mn, Zr, Ce and their mixtures and more particularly the transition metal is selected from Fe and Mn.

**9.** Composite particles according to any one of claims 1 to 8, wherein the metal-doped inorganic UV filter is selected from Fe-doped $TiO_2$ particles and Mn-doped $TiO_2$ particles.

**10.** Composite particles according to any one of claims 1 to 9, containing a core comprising at least a porous or non-porous crosslinked polymethyl methacrylate polymer; the said core being covered at least partially by at least one layer of Fe-doped $TiO_2$ particles.

**11.** Composite particles according to any one of claims 1 to 10, containing a core comprising at least a porous or non-porous crosslinked polymethyl methacrylate polymer; the said core being covered at least partially by at least one layer of Mn-doped $TiO_2$ particles.

**12.** Composition containing, in a physiologically acceptable medium, at least the composite particles as defined in any one of preceding claims.

**13.** Composition according to claim 12 for use in cosmetic method for caring for and/or making up a keratinous substance.

**14.** Composition according to claim 12 for use in cosmetic method for limiting the darkening of the skin and improving the colour and uniformity of the complexion.

**15.** Composition according to claim 12 for use in cosmetic method for treating aging of a keratinous substance.

**Patentansprüche**

1. Verbundteilchen umfassend:

   i) eine Matrix, die mindestens ein anorganisches und/oder mindestens ein organisches Material enthält, und
   ii) mindestens einen anorganischen UV-Filter, der durch mindestens ein Übergangsmetall dotiert ist, wobei die Verbundteilchen kugelförmige Teilchen sind, die einen Kern enthalten, der mindestens ein organisches und/oder ein anorganisches Material umfasst, wobei der Kern wenigstens teilweise mit mindestens einer Schicht eines anorganischen UV-Filters bedeckt ist, der durch mindestens ein Übergangsmetall dotiert ist.

2. Verbundteilchen nach Anspruch 1, die eine mittlere Größe von 0,1 bis 30 μm, vorzugsweise von 0,1 bis 20 μm und mehr bevorzugt von 0,1 bis 10 μm haben.

3. Verbundteilchen nach Anspruch 1 oder 2, **dadurch gekennzeichnet, dass**

   - das anorganische Material aus der aus Glas, Siliciumdioxid und Aluminiumoxid sowie Mischungen davon bestehenden Gruppe ausgewählt ist,
   - das organische Material aus der aus Cellulose, Poly(meth)acrylaten, Polyamiden, Siliconen, Polyurethanen, Polyethylenen, Polypropylenen, Polystyrolen, Polycaprolactamen, Polysacchariden, Polypeptiden, Polyvinylen, Wachsen, Polyestern und Polyethern sowie Mischungen davon bestehenden Gruppe ausgewählt ist.

4. Verbundteilchen nach einem der Ansprüche 1 bis 3, **dadurch gekennzeichnet, dass** der Kern poröse oder nicht poröse kugelförmige Teilchen eines vernetzten Polymethylmethacrylatpolymers enthält.

5. Verbundteilchen nach einem der Ansprüche 1 bis 4, wobei Teilchen des anorganischen UV-Filters aus Metalloxidteilchen bestehen und vorzugsweise aus Zinkoxidteilchen (ZnO), Titandioxidteilchen ($TiO_2$), Eisenoxidteilchen (FeO) und ihren Mischungen und mehr bevorzugt aus $TiO_2$-Teilchen ausgewählt sind.

6. Verbundteilchen nach einem der Ansprüche 1 bis 5, wobei Teilchen des anorganischen UV-Filters eine mittlere elementare Größe von 0,001 bis 0,2 μm haben und vorteilhafterweise eine mittlere elementare Größe von 0,01 bis 0,15 μm haben.

7. Verbundteilchen nach einem der Ansprüche 1 bis 6, wobei der Anteil des metalldotierten anorganischen UV-Filters von 5 bis 80 Gew.-% beträgt, mehr bevorzugt von 5 bis 60 Gew.-%, bezogen auf das Gesamtgewicht der Verbundteilchen .

8. Verbundteilchen nach einem der Ansprüche 1 bis 7, wobei Teilchen des anorganischen UV-Filters durch mindestens ein Übergangsmetall dotiert sind, das aus Fe, Zn, Mn, Zr, Ce und ihren Mischungen ausgewählt ist, und insbesondere ist das Übergangsmetall aus Fe und Mn ausgewählt.

9. Verbundteilchen nach einem der Ansprüche 1 bis 8, wobei der metalldotierte anorganische UV-Filter aus Fe-dotierten $TiO_2$-Teilchen und Mn-dotierten $TiO_2$-Teilchen ausgewählt ist.

10. Verbundteilchen nach einem der Ansprüche 1 bis 9, die einen Kern enthalten, der mindestens ein poröses oder nicht poröses vernetztes Polymethylmethacrylatpolymer umfasst; wobei der Kern wenigstens teilweise von mindestens einer Schicht aus Fe-dotierten $TiO_2$-Teilchen bedeckt ist.

11. Verbundteilchen nach einem der Ansprüche 1 bis 10, die einen Kern enthalten, der mindestens ein poröses oder nicht poröses vernetztes Polymethylmethacrylatpolymer umfasst; wobei der Kern wenigstens teilweise von mindestens einer Schicht aus Mn-dotierten $TiO_2$-Teilchen bedeckt ist.

12. Zusammensetzung, die in einem physiologisch akzeptablen Medium mindestens die Verbundteilchen nach einem der vorhergehenden Ansprüche enthält.

13. Zusammensetzung nach Anspruch 12 zur Verwendung bei einem kosmetischen Verfahren zur Pflege und/oder zum Schminken einer Keratinsubstanz.

14. Zusammensetzung nach Anspruch 12 zur Verwendung bei einem kosmetischen Verfahren zur Begrenzung des

Dunklerwerdens der Haut und zur Verbesserung der Farbe und Gleichmäßigkeit des Teints.

**15.** Zusammensetzung nach Anspruch 12 zur Verwendung bei einem kosmetischen Verfahren zur Behandlung des Alterns einer Keratinsubstanz.

## Revendications

**1.** Particules composites comprenant

i) une matrice comportant au moins un matériau inorganique et/ou au moins un matériau organique,
ii) et au moins un filtre UV inorganique qui est dopé avec au moins un métal de transition,

lesquelles particules composites sont des particules sphériques qui comportent un cœur comprenant au moins un matériau inorganique et/ou au moins un matériau organique, lequel cœur est recouvert, au moins en partie, par au moins une couche de filtre UV inorganique qui est dopé avec au moins un métal de transition.

**2.** Particules composites conformes à la revendication 1, dont la taille moyenne vaut de 0,1 à 30 μm, de préférence de 0,1 à 20 μm, et mieux encore de 0,1 à 10 μm.

**3.** Particules composites conformes à la revendication 1 ou 2, **caractérisées en ce que**

- le matériau inorganique est choisi dans l'ensemble formé par les suivants : verre, silice et oxyde d'aluminium, et leurs mélanges,
- et le matériau organique est choisi dans l'ensemble formé par les suivants : cellulose, poly(méth)acrylates, polyamides, silicones, polyuréthanes, polyéthylènes, polypropylènes, polystyrènes, polycaprolactames, polysaccharides, polypeptides, polyvinyliques, cires, polyesters et polyéthers, et leurs mélanges.

**4.** Particules composites conformes à l'une des revendications 1 à 3, **caractérisées en ce que** le cœur contient des particules sphériques, poreuses ou non poreuses, d'un polymère poly(méthacrylate de méthyle) réticulé.

**5.** Particules composites conformes à l'une des revendications 1 à 4, dans lesquelles les particules du filtre UV inorganique sont constituées de particules d'oxyde de métal et sont de préférence choisies parmi des particules d'oxyde zinc ZnO, des particules de dioxyde de titane $TiO_2$, des particules d'oxyde de fer FeO et des mélanges de telles particules, et mieux encore, sont des particules de $TiO_2$.

**6.** Particules composites conformes à l'une des revendications 1 à 5, dans lesquelles les particules du filtre UV inorganique présentent une taille moyenne élémentaire de 0,001 à 0,2 μm, et avantageusement, présentent une taille moyenne élémentaire de 0,01 à 0,15 μm.

**7.** Particules composites conformes à l'une des revendications 1 à 6, dans lesquelles la proportion du filtre UV inorganique dopé avec un métal vaut de 5 à 80 % en poids, et de préférence, de 5 à 60 %, en poids rapporté au poids total des particules composites.

**8.** Particules composites conformes à l'une des revendications 1 à 7, dans lesquelles les particules du filtre UV inorganique sont dopées avec au moins un métal de transition choisi parmi les fer, zinc, manganèse, zirconium et cérium et leurs mélanges, étant entendu que ce métal de transition est plus spécialement choisi parmi du fer et du manganèse.

**9.** Particules composites conformes à l'une des revendications 1 à 8, dans lesquelles le filtre UV inorganique dopé avec un métal est choisi parmi des particules de dioxyde de titane Ti02 dopées au fer et des particules de dioxyde de titane $TiO_2$ dopées au manganèse.

**10.** Particules composites conformes à l'une des revendications 1 à 9, qui comportent un cœur comprenant au moins un polymère poly(méthacrylate de méthyle) réticulé, poreux ou non poreux, lequel cœur est recouvert, au moins en partie, par au moins une couche de particules de dioxyde de titane $TiO_2$ dopées au fer.

**11.** Particules composites conformes à l'une des revendications 1 à 10, qui comportent un cœur comprenant au moins

un polymère poly(méthacrylate de méthyle) réticulé, poreux ou non poreux, lequel cœur est recouvert, au moins en partie, par au moins une couche de particules de dioxyde de titane $TiO_2$ dopées au manganèse.

**12.** Composition contenant, dans un milieu physiologiquement admissible, au moins des particules composites telles que définies dans l'une des revendications précédentes.

**13.** Composition conforme à la revendication 12 pour utilisation dans un procédé cosmétique pour le soin et/ou le maquillage d'une substance kératineuse.

**14.** Composition conforme à la revendication 12 pour utilisation dans un procédé cosmétique pour limiter le brunissement de la peau et améliorer la couleur et l'uniformité du teint.

**15.** Composition conforme à la revendication 12 pour utilisation dans un procédé cosmétique pour traiter le vieillissement d'une substance kératineuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012105723 A
- WO 2012105060 A
- WO 2012105059 A
- WO 2011016140 A
- WO 2011016143 A
- FR 2971152
- WO 2012104160 A
- WO 2012104161 A
- WO 9960994 A
- WO 0140114 A
- WO 2005002538 A
- WO 9206778 A
- EP 1069142 A
- FR 2315991
- FR 2416008
- WO 2007068371 A, Cognis
- WO 2008155059 A
- US 5624663 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 04006878 A
- WO 05058269 A
- WO 06032741 A
- EP 0823418 A
- EP 1341752 A
- EP 0841341 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0518773 A
- US 4077441 A
- US 4850517 A


### Non-patent literature cited in the description


- *Mat. Sci.,* 1997, vol. 36, 6001-6008
- **BANGHAM ; STANDISH ; WATKINS.** *J. Mol. Biol.,* 1965, vol. 13, 238
- *J. Soc. Cosm. Chem.,* 1954, vol. 5, 249-256
- HLB Index. McCutcheon's Emulsifiers & Detergents. MC Publishing Company, 1998
- Symmetrical Triazine Derivatives. IP.COM Journal. IP.COM INC, 20 September 2004
- *Cosmetics & Toiletries,* February 1990, vol. 105, 53-64
- **B.L. DIFFEY.** *J. Soc. Cosmet. Chem.,* 1989, vol. 40, 127-133
- Measurement Standards for UVA protection efficacy. *Japan Cosmetic Industry Association Technical Bulletin,* 21 November 1995